# EUROPÄISCHE PATENTSCHRIFT

(11) **EP 0 749 973 B1**
(45) Veröffentlichungstag und Bekanntmachung des Hinweises auf die Patenterteilung: **14.11.2001**
(21) Anmeldenummer: 96109252.5
(22) Anmeldetag: 10.06.1996
(51) Int. Cl.: C07F 9/50

(54) **Neue Bisphosphine als Katalysatoren für asymmetrische Reaktionen**
Novel bisphosphines as catalysts for asymmetric reactions
Nouvelles bisphosphines comme catalyseurs de réactions asymmétriques

(30) Priorität: 20.06.1995 DE 19522293
(43) Veröffentlichungstag der Anmeldung: 27.12.1996
(73) Patentinhaber: BAYER AG, 51368 Leverkusen (DE)
(72) Erfinder: Laue, Christian, Dr., 40789 Monheim (DE); Schröder, Georg, Dr., 51375 Leverkusen (DE); Arlt, Dieter, Prof. Dr., 51065 Köln (DE)

(56) Entgegenhaltungen:
- EP-A- 0 104 375
- EP-A- 0 398 132
- EP-A- 0 643 065
- SYNLETT, Nr. 11, 1991, STUTTGART DE, Seiten 827-829, XP000604849 M. MURATA: "Synthesis of Atropisomeric Biphenylphosphine,6,2'-Bis(diphenylphosphi no)-3-methoxy-2,4-dimethyl-4',6'-bis(tri- fluoromethyl)-1,1'-biphenyl"

## Beschreibung

Die Erfindung betrifft neue enantiomerenreine Bisphosphine, Verfahren zu ihrer Herstellung und ihre Verwendung in Metallkomplexen als Katalysatoren für asymmetrische Reaktionen, insbesondere asymmetrische Hydrierungen.

Es ist bereits bekannt, Komplexe von bestimmten Bisphosphinen mit Metallen der Gruppe VIII des Periodensystems für asymmetrische Hydrierungen und enantioselektive Wasserstoffverschiebungen einzusetzen (vgl. R. Schmid et al. Helv. Chim. Acta, 74, 370 (1991)).

In EP-A-104 375 wird ein Bisphosphin beansprucht, das sich von den erfindungsgemäßen Phosphinen dadurch unterscheidet, das dort anstelle des Chlor-Atoms ein Fluor steht. Von diesen bekannten Fluor-Verbindungen war jedoch anzunehmen, das sie wegen der hohen Elektronegativität des Fluors ähnlich schlecht reagieren wie das mit elektronegativen Substituenten versehene BIFUP (K. Achiwa Synlett, 1991, 827). Dort steht auf Seite 828 (siehe auch Tabelle 1), daß der Ligand wegen der elektronegativen Gruppen nur schlechten Umsatz erbringt. Auch verschlechterte sich die Stereoselektivität; d.h. der Anteil der Minderkomponente stieg von <0,5 %, die von bisher bekannten anderen Bisphosphine erreicht werden, auf 2,5 % (= 95 % e.e.).

Der erfindungsgemäße Ligand hat nun statt dem Halogen Fluor das Halogen Chlor in dieser Position und zeigt z.B. in Form von Ru-Komplexen bei der Hydrierung von Acetessigestermethylester ausgesprochen gute Selektivitäten von >99 % e.e., was aufgrund des obigen Vorurteils nicht zu erwarten war.

Die erfindungsgemäßen Liganden sind weiterhin beispielsweise in der Lage, in Form ihrer Ruthenium-Komplexe die Hydrierung von 2-(3-Benzyl-phenyl)-Propensäure zur entsprechenden Propansäure (=S-Ketoprofen-Derivat) mit einer hohen Selektivität zu katalysieren.

In DE-A-4 330 730 sind bereits Liganden beschrieben, die bei der Hydrierung obiger Propensäure-Derivate eine Selektivität von 88 % e.e. liefern. Die dort benutzten Liganden lassen sich jedoch nur mit schlechten Enantiomerenreinheiten durch Kristallisation in die Enantiomeren spalten, weshalb man, wie im obigen Patent beschrieben, auf die aufwendige Racematspaltung durch Chromatographie an chiralen Phasen angewiesen ist. Die erfindungsgemäßen Liganden lassen sich hingegen leicht durch Kristallisation in die Enantiomere spalten.

Die Erfindung betrifft enantiomerenreine Bisphosphine der allgemeinen Formel (I) 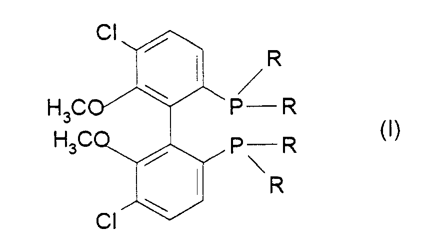 in welcher
- R: jeweils für eine Phenylgruppe steht, die ihrerseits mit 1 bis 3 Substituenten aus der Gruppe OR¹, R¹, Nitro, NH₂, NHR¹ oder NR¹₂ substituiert sein kann, wobei R¹ eine Alkylgruppe mit bis zu 6, vorzugsweise bis zu 4 Kohlenstoffatomen darstellt, oder
- R: eine Alkylgruppe mit bis zu 7, vorzugsweise mit bis zu 4 C-Atomen oder eine Cyclo-Alkylgruppe mit 3 bis 7, vorzugsweise 5 oder 6 C-Atomen darstellt.

Besonders bevorzugt sind enantiomerenreine Bisphosphine der allgemeinen Formel (I), in welcher R für Phenyl steht.

Die Bisphosphine der allgemeinen Formel (I) können hergestellt werden, indem man
a) 5-Br-2-Cl-Anisol mit Phosphoroxychloriden der allgemeinen Formel (II) 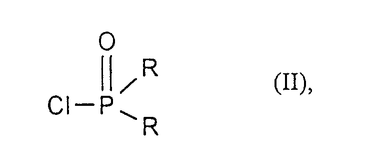 in welcher
   - R: die oben angegebene Bedeutung hat
   nach üblichen Methoden, nach einer selektiven Mono-Metallierung von Brom-Chlor-Anisol, vorzugsweise durch eine Grignard Reaktion, zu Verbindungen der allgemeinen Formel (III) 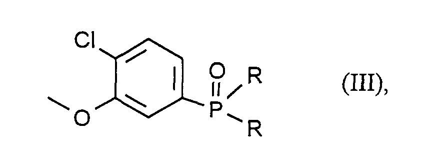 in welcher
   - R: die oben angegebene Bedeutung hat,
   umsetzt und diese in 6-Position nach Methoden der ortho-Metallierung metalliert, vorzugsweise mit Lithiumamid und anschließend mit Iod zu Verbindungen der allgemeinen Formel (IV) 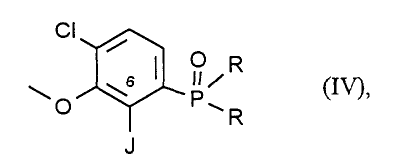 in welcher
   - R: die oben angegebene Bedeutung hat,
   umsetzt und anschließend nach üblichen Methoden zu racemischen Verbindungen der allgemeinen Formel (V) 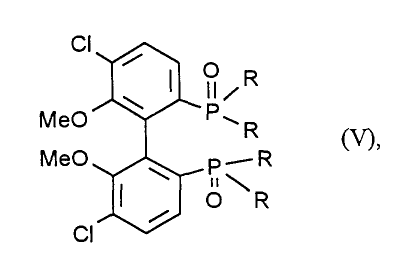 in welcher
   - R: die oben angegebene Bedeutung hat,
   dimerisiert und diese Phosphinoxide durch Kristallisation mit enantiomerenreinen Mono- oder Dicarbonsäuren in ihre Enantiomere auftrennt und die getrennten Enantiomere zu Verbindungen der Formel (I) reduziert, oder
b) indem man alternativ Verbindungen der allgemeinen Formel (III) herstellt, indem man 5-Br-2-Cl-Anisol metalliert, z.B. über das Mono-Grignard-Reagenz, dann mit Phosphinchloriden der allgemeinen Formel (VI) 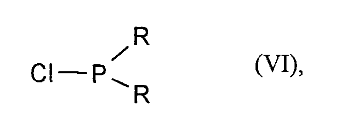 in welcher
   - R: die oben angegebene Bedeutung hat,
   umsetzt und anschließend nach üblichen Methoden oxidiert.

Die erfindungsgemäßen Bisphosphine der Formel (I) bilden Komplexe mit Übergangsmetallen wie etwa Metallen der Gruppe VIII, insbesondere mit Ruthenium, Rhodium und Iridium, welche als Katalysatoren bei asymmetrischen Hydrierungen und auch für enantioselektive Wasserstoffverschiebungen in prochiralen, allylischen Systemen verwendbar sind. Für die erwähnten Hydrierungen sind Ruthenium-, Iridium- und Rhodium-Komplexe bevorzugt, während für Isomerisierungen Rhodium-Komplexe bevorzugt sind. Diese Katalysatoren, d.h. die Komplexe aus einem Metall der Gruppe VIII und den Phosphorverbindungen der Formel (I) sind neu und ebenfalls Gegenstand der vorliegenden Erfindung.

Die in Frage stehenden Komplexe können in an sich bekannter Weise hergestellt werden, z.B. indem man eine Verbindung der Formel (I) mit einer Verbindung, welche ein Metall der Gruppe VIII abgeben kann, in einem geeigneten, inerten organischen oder wäßrigen Lösungsmittel umsetzt. Als geeignete, z.B. Rhodium abgebende Verbindungen können beispielsweise genannt werden, organische Rhodium-Komplexe mit Ethylen, Propylen und dergleichen, sowie mit bis-Olefinen, z.B. 1,5-Cyclooctadien, 1,5-Hexadien, Bicyclo(2.2.1)hepta-2,5-dien oder mit weiteren Dienen, welche mit Rhodium leicht lösliche Komplexe bilden.

Bevorzugte, Rhodium abgebende Verbindungen sind z.B. Di-chloro-bis-1,5-cyclooctadien)dirhodium, Di-chloro-bis(-norbornadien)dirhodium, Bis- 1,5-cyclooctadien)-rhodium-tetrafluorborat oder Bis-(cyclooctadien)rhodium-perchlorat. Als Iridium abgebende Verbindung kann beispielsweise Di-chloro-bis-1,5-cyclooctadien)diiridium genannt werden.

Von besonderem Interesse sind Rutheniumkomplexe mit Bisphosphinen der allgemeinen Formel (I). Als typisch aber nicht als Einschränkung seien die Rutheniumkomplexe der folgenden Formeln (VII) bis (XIII) genannt.

### Beispiele für typische Rutheniumkomplexe

Ru₂Cl₄B₂(S) (VII)

[Ru Hal Q B]^{⊕} Y ^{⊖} (VIII)

Ru Bₙ OOCR³OOCR⁴ (IX)

[Ru Hₓ Bₙ]^{m⊕} Yₘ ^{⊖} (X)

[Ru Hal (PR⁵ ₂R⁶)B]⁽²⁺⁾ Hal₂ ^{⊖} (XI)

[Ru H Hal B₂] (XII)

[B Ru (acac)₂] (XIII)

[B Ru Y₂] (XIV)

worin:
- acac: Acetylacetonat
- B: für ein Bisphosphin der allgemeinen Formel (I) steht,
- Hal: für Halogen, insbesondere für Iod, Chlor oder Brom steht,
- R³ und R⁴: gleich oder verschieden sind und für Alkyl mit bis zu 9 C-Atomen, vorzugsweise bis zu 4 C-Atomen, welches gegebenenfalls substituiert ist durch Halogen, insbesondere Fluor, Chlor oder Brom oder für Phenyl steht, welches gegebenenfalls durch Alkyl mit 1 bis 4 C-Atomen substituiert ist oder für eine α-Aminoalkylsäure mit vorzugsweise bis zu 4 C-Atomen stehen,
oder
gemeinsam eine Alkylidengruppe mit bis zu 4 C-Atomen bilden,
- R⁵ und R⁶: jeweils gleich oder verschieden sind und für gegebenenfalls substituiertes Phenyl stehen, vorzugsweise substituiert durch Alkyl mit 1 bis 4 C-Atomen oder Halogen,
- Y: für Cl, Br, J, ClO₄, BF₄ oder PF₆ steht,
- Q: für einen unsubstituierten oder substituierten Benzolring wie p-Cymol steht,
- S: für ein tertiäres Amin wie z.B. Triethylamin, Tri-n-Butylamin oder Pyridin steht,
- n und m: stehen jeweils für 1 oder 2,
- x: steht für 0 oder 1,
wobei in Formel (X) ein n für 1 und m für 2 steht, wenn x = 0 bedeutet,
und n für 2 und m für 1 steht, wenn x = 1 bedeutet.

Die Komplexe der Formeln (VII) bis (XIII) können nach an sich bekannten Methoden hergestellt werden.

Die Komplexe der Formeln (VII) und (XII) lassen sich z.B. in analoger Weise gemäß den Verfahren herstellen, die in EP-A-174 057 oder in Chem. Comm. 922 (1985) beschrieben werden.

Die Komplexe der allgemeinen Formel (VIII) ergeben sich z.B. durch Umsetzung von bekannten Rutheniumkomplexen [RuHal₂Q]₂ mit Bisphosphinen der allgemeinen Formel (I) in inerten organischen Lösungsmitteln, wie z.B. in EP-A-366 390 beschrieben.

Komplexe der allgemeinen Formel (IX), n = 1 können z.B. erhalten werden nach Verfahren, die in EP-A-245 959 angegeben sind, indem man Komplexe der allgemeinen Formel (VII) mit entsprechenden Carbonsäuren, vorzugsweise in alkoholischen Lösungsmitteln umsetzt.

Komplexe der Formeln (IX) n = 2 und mit n = 1 und R³, R⁴ = CF₃ können nach den in EP-A-272 787 angegebenen Verfahren hergestellt werden.

Die Komplexe der allgemeinen Formel (X) können hergestellt werden analog dem Verfahren gemäß EP-A-256 634.

Die Komplexe der allgemeinen Formel (XI) können hergestellt werden analog dem Verfahren gemäß EP-A-470 756 durch Umsetzung der dort beschriebenen Ru-Vorstufen mit den erfindungsgemäßen Bisphosphinen der allgemeinen Formel (I).

Komplexe der Formel (XIII) können analog den in P. Stahly et al., Organo-mettalics 1993, 1467 ff. angegebenen Verfahren dargestellt werden.

Komplexe der Formel (XIV) können analog der in J.A.C.S. 1987, 109, 5856 bis 5858 angegebenen Verfahren hergestellt werden.

Die erfindungsgemäßen Bisphosphine in Form ihrer Komplexe mit Metallen der Gruppe VIII und insbesondere mit Ruthenium sind verwendbar für asymmetrische Hydrierungen. Geeignete Substrate sind substituierte oder unsubstituierte α- oder β-Ketoester oder α- oder β-Keto-Amide, α- oder β-Amino- oder α- oder β-Hydroxy-Ketone und Acetamidozimtsäurederivate.

Weiterhin geeignet sind Acrylsäuren, insbesondere 2-Arylpropensäuren wie z.B. 2-(6'-Methoxy-2'-naphthyl)-propensäure, 2-(4-Isobutyl)-propensäure und 2-(3-Benzylphenyl)-propensäure und ihre Salze, z.B.mit tertiären Aminen.

Bei der Durchführung derartiger Hydrierungen können diese Komplexe zuerst hergestellt und dann zu einer Lösung der zu hydrierenden Substanz gegeben werden. Alternativ können sie jedoch auch in situ hergestellt werden, z.B. auch in Gegenwart einer zu hydrierenden Substanz.

Die asymmetrische Hydrierung kann in einem geeigneten, unter den Reaktionsbedingungen inerten organischen Lösungsmitteln erfolgen. Als derartige Lösungsmittel können insbesondere genannt werden, niedere Alkohole wie z.B. Methanol oder Ethanol, oder Gemische derartiger Alkohole mit halogenierten Kohlenwasserstoffen wie Methylenchlorid, Chloroform und dergleichen, oder mit cyclischen Ethern wie Tetrahydrofuran oder Dioxan, und dergleichen.

Das Verhältnis von den Metallen zu Bisphosphinen der allgemeinen Formel (I) liegt zweckmäßig zwischen etwa 0,5 und etwa 2 Mol, vorzugsweise bei etwa 1 Mol Ruthenium pro Mol Bisphosphin-Ligand. Das Verhältnis von Metall in den Komplexen zu den hydrierenden Substanzen liegt zweckmäßig zwischen etwa 0,0005 und 1 Mol-%, vorzugsweise zwischen etwa 0,005 und 0,6 Mol-%.

Die asymmetrische Hydrierung mit den erfindungsgemäßen Komplexen erfolgt zweckmäßig bei einer Temperatur von etwa 0°C bis etwa 100°C, in Abhängigkeit des verwendeten Substrates. Diese Hydrierung erfolgt zweckmäßig auch unter einem Druck, vorzugsweise bei einem Druck von etwa 5 bis etwa 200 bar, besonders bevorzugt von etwa 40 bis etwa 140 bar.

Weiterhin sind die erfindungsgemäßen Bisphosphinkomplexe als Katalysatoren verwendbar für enantioselektive Wasserstoffverschiebungen in prochiralen allylischen Systemen. Besonders interessant sind sie z.B. im Zusammenhang mit der Herstellung von optisch aktiven Verbindungen der allgemeinen Formel (XV) 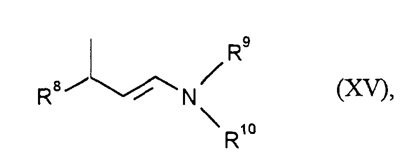 worin
- R⁸: geschütztes Hydroxymethyl oder einen Rest der Formel 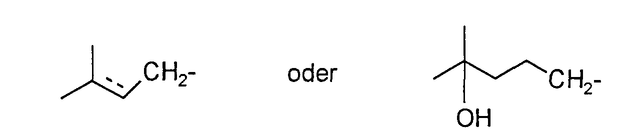 darstellt, worin die gestrichelte Linie eine zusätzliche Bindung darstellen kann, und
- R⁹ und R¹⁰: gleich oder verschieden sind und niederes Alkyl (1-7 C-Atome) bedeuten,
ausgehend von Verbindungen der allgemeinen Formel (XVI) 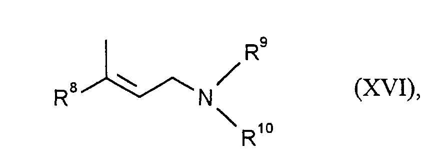 worin
- R⁸, R⁹ und R¹⁰: die obige Bedeutung haben.

Die Verbindungen (XV) bzw. die daraus durch Hydrolyse erhaltenen Aldehyde, sowie die von letzteren abgeleiteten Säuren und Alkohole sind z.B. von Interesse als Zwischenprodukte bei der Synthese der Seitenketten der Vitamine E und K_{I}.

Zur Durchführung der erwähnten Wasserstoffverschiebungen können die Phosphorverbindungen der Formel (I) als solche in einer Lösung einer zu behandelnden Verbindung mit einer z.B. Rhodium oder Iridium abgebenden Verbindung in Kontakt gebracht werden. Andererseits können die Phosphorverbindungen der Formel (I) zunächst in einem geeigneten Lösungsmittel mit einer z.B. Rhodium oder Iridium abgebenden Verbindung zu dem entsprechenden Katalysator-Komplex umgesetzt werden, und dieser dann zu einer Lösung einer zu behandelnden Verbindung gegeben werden, wobei die letztere Methode bevorzugt ist.

Sowohl die Umsetzung der Phosphorverbindungen der Formel (I) mit einer z.B. Rhodium oder Iridium abgebenden Verbindung, wie auch die erwähnten Wasserstoffverschiebungen können in geeigneten, unter den Reaktionsbedingungen inerten organischen Lösungsmitteln durchgeführt werden. Als solche seien insbesondere genannt niedere Alkanole wie z.B. Methanol oder Ethanol, aromatische Kohlenwasserstoffe wie Benzol oder Toluol, cyclische Ether wie Tetrahydrofuran oder Dioxan, Ester wie z.B. Essigester, oder auch Gemische hiervon, und dergleichen. Weiterhin können die Komplexbildungen auch in wäßrigem Medium oder in Dichlormethan durchgeführt werden.

Das Verhältnis zwischen z.B. Rhodium oder Iridium und den Liganden der Formel (I) liegt zweckmäßig zwischen etwa 0,05 und etwa 5 Mol, vorzugsweise zwischen etwa 0,5 und etwa 2 Mol Metall pro Mol Ligand der Formel (I).

Die Menge an Metall, in den Komplexen mit den Liganden der Formel (I), bezogen auf die zwecks Wasserstoffverschiebung zu behandelnden Verbindungen, liegt vorzugsweise zwischen etwa 0,005 und etwa 0,5 Mol-%, insbesondere zwischen etwa 0,01 und etwa 0,2 Mol-%.

Die erwähnten Wasserstoffverschiebungen unter Verwendung von Metall-Komplexen mit den Liganden der Formel (I) können zweckmäßig in einem inerten, organischen Lösungsmittel und bei einer Temperatur von etwa Raumtemperatur bis etwa 130°C durchgeführt werden. Diese Reaktion erfolgt vorzugsweise bei erhöhter Temperatur, d.h. je nach verwendetem Lösungsmittel entweder bei Rückflußtemperatur des Reaktionsgemisches oder in einem geschlossenen Gefäß unter Druck.

### Experimenteller Teil:

Im folgenden werden folgende Abkürzungen verwendet:
- cym:: 4-Isopropyltoluol
- THF:: Tetrahydrofuran
- DMF:: Dimethylformamid
- TBME:: tert-Butyl-methylether
- HPLC:: high pressure liquid chromatography
- LDA:: Lithium-diisopropyl-amid

### A) Herstellung der Bisphosphine

1a) Diphenyl-(4-chlor-3-methoxy-phenyl)-phosphinoxid (Formel III)
   9,05 g Mg-Späne werden unter Argon mit einer Spatelspitze Iod erhitzt, bis Ioddämpfe entstehen und es wird 5 Minuten trocken gerührt. Anschließend werden 350 ml THF (p.a.) zugegeben und auf Siedetemperatur erhitzt. 75 g 5-Brom-2-chlor-anisol in 360 ml THF werden unter Rückfluß in einer 1/2 Stunde zugegeben, wobei man sich bei Zugabe der ersten Mengen vom Anspringen der Reaktion überzeugt. Es wird noch 40 Minuten unter Rückfluß nachgerührt.
   Es wird auf 0°C gekühlt und 88,5 g Diphenylphosphinsäurechlorid in 450 ml THF werden in 25 Minuten unter Eiskühlung bei 0 bis 5°C zugetropft. Es wird 2 Stunden bei Raumtemperatur gerührt. Bei 0 bis 15°C wird in 15 Minuten 450 ml 1 N HCl zugetropft und es wird 15 Minuten gut gerührt. Die organische Phase wird abgetrennt und die wäßrige Phase wird zweimal mit je 0,5 1 Methylenchlorid extrahiert und die vereinigten organischen Phasen werden mit je 0,5 l 1 N NaOH, mit Wasser und gesättigter NaCl-Lösung gewaschen mit MgSO₄ getrocknet und eingeengt. 120 g Rohprodukt werden mit 400 ml TBME ausgerührt, bei Raumtemperatur auf ca. 150 ml eingeengt, 2 Stunden gerührt und filtriert.
   Ausbeute: 81 g (70 % Ausbeute)
   m.p.: 104 bis 107°C
1b) Diphenyl-(4-chlor-3-methoxy-phenyl)-phosphinoxid (Formel (III)
   1,31 g Mg-Späne werden unter Argon mit einer Spatelspitze Iod mit Heißluft erhitzt bis Ioddämpfe entstehen und es wird 5 Minuten trocken gerührt. Anschließend werden 50 ml THF zugegeben und auf Siedetemperatur erhitzt. 10 g 5-Brom-2-chlor-anisol in 50 ml THF werden unter Rückfluß in einer 1/2 Stunde zugegeben wobei man sich bei Zugabe der ersten Mengen vom Anspringen der Reaktion überzeugt. Es wird noch 40 Minuten unter Rückfluß nachgerührt.
   Es wird auf 0°C gekühlt und 9,9 g Diphenylphosphinsäurechlorid in 50 ml THF werden in 25 Minuten unter Eiskühlung bei 0 bis 5°C zugetropft. Es wird 2 Stunden bei Raumtemperatur gerührt. Unter Kühlung werden 2,8 g Methanol zugegeben und der Ansatz am Rotationsverdampfer zur Trockene eingeengt. Der Rückstand wird mit 200 l Aceton aufgenommen und 13,1 g aq. Wasserstoffperoxid-Lösung zugegeben. Nach 45 Minuten Rühren bei Raumtemperatur und der Zugabe von 200 ml Wasser und 135 ml gesättigte wäßrige Natriumdithionit-Lösung wird das Aceton im Vakuum entfernt und die zurückbleibende Lösung mit Methylenchlorid extrahiert. Anschließend wird die organische Phase mit Wasser, gesättigte NaCl-Lösung extrahiert, mit MgSO₄ getrocknet und eingeengt. Der Rückstand wird mit TBME bei 0°C ausgerührt und filtriert.
   Ausbeute: 5,91 g (40 %)
   m.p.: 104 bis 107°C
2) Diphenyl-(2-iodo-4-chlor-3-methoxy-phenyl)-phosphinoxid (Formel (IV))
   Zu einer Lösung von 64,2 g Diphenyl-(4-chlor-3-methoxy-phenyl)-phosphinoxid in 930 ml THF werden bei -70°C in 5 Minuten 112 ml (LDA in THF; 2M) zugegeben. Die Lösung wird auf 0°C aufgewärmt und 5 Minuten bei dieser Temperatur belassen. Anschließend wird bei -76°C eine Lösung von 56,8 g J₂ in 670 ml THF in 75 Minuten zugetropft, noch 5 Minuten bei dieser Temperatur gerührt, in 45 Minuten auf Raumtemperatur erwärmt und noch 1 Stunde bei dieser Temperatur gerührt.
   Zur Aufarbeitung wird mit 320 ml Na₂SO₃-Lösung und 200 ml Wasser versetzt und mit Essigester extrahiert. Die organische Phase wird mit gesättigter NaCl-Lösung gewaschen, mit MgSO₄ getrocknet und vom Lösungsmittel befreit.
   Roh-Ausbeute: 90,6 g (103 Gew.-%)
   Reinheit nach ¹H-NMR: ca. 70 %
3) rac.-(5,5'-Dichloro-6,6'-dimethoxy-biphenyl-2,2'-diyl)-bis-(diphenylphosphinoxid (Formel (V))
   Eine Mischung von 90,5 g rohem Diphenyl-(2-iodo-4-chlor-3-methoxyphenyl)-phosphinoxid und 37,1 g Cu-Pulver werden mit 395 ml DMF 16 Stunden bei 140°C unter Argon gekocht. Es wird heiß über eine Glasfritte abgesaugt und mit insgesamt ca. 100 ml warmen DMF nachgewaschen. Das Filtrat wird vom Lösungsmittel befreit und mit 900 ml TBME ausgerührt.
   Ausbeute: 39,1 g
   m.p.: 175°C (Zersetzung).
4) (-)-(5,5'-Dichloro-6,6'-dimethoxy-biphenyl-2,2'-diyl)-bis-(diphenyl-phosphinoxid (Formel (V))
   Zu einer Lösung von 13,5 g (5,5'-Dichloro-6,6'-dimethoxy-biphenyl-2,2'-diyl)-bis-(diphenyl-phosphinoxid) in 200 ml Methylenchlorid wird bei Raumtemperatur eine Lösung von 7,07 g wasserfreie (-)-Dibenzoylweinsäure in 110 ml Essigester zugetropft. Die entstehende Suspension wird über Nacht gerührt und filtriert. Der Rückstand wird getrocknet und das Filtrat wird vom Lösungsmittel befreit.
   Rohauswaage Salz Kristalle: 8,05 g (39 Gew.-%)
   Rohauswaage Salz Filtrat: 11,61 g (56 Gew.-%)
   Die Kristalle wurden in 100 ml Methylenchlorid aufgenommen und zweimal mit je 25 ml 1N NaOH-Lösung extrahiert. Die organische Phase wird mit 1N HCl, 1N NaOH und gesättigter NaCl-Lösung extrahiert und mit MgSO₄ getrocknet.
   Ausbeute Kristalle nach Aufarbeitung: 5,33 g (39 % Ausbeute)
   Enantiomerenreinheit: 99,4 % e.e.
   Die Kontrolle der Enantiomerenreinheit erfolgt durch analytische HPLC. Als Laufmittel wird n-Heptan/THF 1:1 verwendet.
   [α]_{D} = -99,7 (c=1, DMF)
   m.p.: 179°C
5) (+)-(5,5'-Dichloro-6,6'-dimethoxy-biphenyl-2,2'-diyl)-bis-(diphenylphosphinoxid) (Formel V)
   Das unter 4) erhaltene Filtrat wird eingeengt und analog zu der Aufarbeitung der Kristalle unter 4) aufgearbeitet.
   Ausbeute Filtrat nach Aufarbeitung: 7,2 g (53 %) Ausbeute
   Enantiomerenreinheit nach HPLC: 84,1 % e.e.
   Zu einer Lösung von 7,0 g rohem, aufgearbeitetem Filtrat (-)-(5,5'-Dichloro-6,6'-dimethoxy-biphenyl-2,2'-diyl)-bis-(diphenyl-phosphinoxid) in 104 ml Methylenchlorid wird bei Raumtemperatur eine Lösung von 3,5 g wasserfreier (+)-Dibenzoylweinsäure in 59 ml Essigester zugetropft. Die entstehende Suspension wird über Nacht gerührt und filtriert. Der Rückstand wird getrocknet und das Filtrat wird vom Lösungsmittel befreit.
   Rohauswaage Salz Kristalle: 7,85 g (38 % bezogen auf rac.-(5,5'-Dichloro-6,6'-dimethoxy-biphenyl-2,2'-diyl)-bis-(diphenyl-phosphinoxid)
   Rohauswaage Salz Filtrat: 2,84 g
   Die Kristalle werden wie oben beschrieben aufgearbeitet.
   Ausbeute Kristalle nach Aufarbeitung: 5,17 g (38 % bezogen auf rac.-(5,5'-Dichloro-6,6'-dimethoxy-biphenyl-2,2'-diyl)-bis-(diphenyl-phosphinoxid)
   Enantiomerenreinheit: >99,9 % e.e. (durch HPLC)
   m.p.: 176°C
   [α]_{D} = + 104, 1 (C=1, DMF)
6) (+)-(5,5'-Dichloro-6,6'-dimethoxy-biphenyl-2,2'-diyl)-bis-(diphenylphosphin) Formel (I)
   5,06 g (+)-(5,5'-Dichloro-6,6'-dimethoxy-biphenyl-2,2'-diyl)-bis-(diphenylphosphinoxid) werden unter Rückfluß in einer Mischung aus 195 ml Xylol absolutem 35,8 ml Tributylamin und 9,1 ml Trichlorsilan 2,5 Stunden gekocht. Zur Aufarbeitung wird die Mischung unter Kühlung langsam mit 72 ml 30 % aq. NaOH versetzt, die organische Phase abgetrennt und danach mit 75 ml TBME extrahiert. Die organischen Phasen werden mit Wasser und gesättiger NaCl-Lösung extrahiert, mit MgSO₄ getrocknet und vom Lösungsmittel befreit. Der Rückstand wird aus 65 ml Ethanol ausgerührt und filtriert.
   Ausbeute: 4,0 g (84 %)
   m.p.: 220 bis 223°C (Zers.)
   [α]_{D} = +55,1 (c=1, CHCl₃)
   Enantiomerenüberschuß: >99,9 % (durch HPLC)
7) (-)-(5,5'-Dichloro-6,6'-dimethoxy-biphenyl-2,2'-diyl)-bis-(diphenyl-phosphin) (Formel (I))
   2,35 g (-)-(5,5'-Dichloro-6,6'-dimethoxy-biphenyl-2,2'-diyl)-bis-(diphenylphosphinoxid) werden analog 6) umgesetzt.
Ausbeute: 1,78 g (80 %)
[α]_{D} = -50,7 (c=1, CHCl₃)
m.p.: 223 bis 225°C
Enantiomerenüberschuß >99,9 % (durch HPLC)

### B) Referenzbeispiel aus DE 4 330 730

1a) Racematspaltung von (Bis-4,4'-dibenzofuran-3-yl)-diphenylphosphinoxid durch Kristallisation
   Zu einer Lösung von 735 mg (Bis-4,4'-dibenzofuran-3-yl)-diphenylphosphinoxid in 44 ml Chloroform wird bei Raumtemperatur eine Lösung von 358 mg wasserfreie (-)(-)-Dibenzoylweinsäure in 29 ml Essigester zugetropft und 30 Minuten unter Rückfluß erhitzt. Anschließend wird 48 Stunden bei Raumtemperatur gerührt und filtriert. Der Rückstand wird getrocknet und das Filtrat wird vom Lösungsmittel befreit.
   Die Kristalle werden in 5 ml Methylenchlorid aufgenommen und zweimal mit je ca. 3 ml 1N NaOH-Lösung extrahiert. Die organische Phase wird 1N HCl, 1N NaOH, gesättigter NaCl-Lösung extrahiert und mit MgSO₄ getrocknet.
   Ausbeute Kristalle nach Aufarbeitung: 47 mg
   Enantiomerenreinheit: 5 % e.e. (nach HPLC)
1b) Zu einer Lösung von 500 mg (Bis-4,4'-dibenzofuran-3-yl)-diphenylphosphinoxid in 7,5 ml Methylenchlorid wird bei Raumtemperatur eine Lösung von 244 mg wasserfreie (-)-Dibenzoylweinsäure in 3,8 ml Essigester zugetropft und 30 Minuten unter Rückfluß erhitzt. Anschließend wird 48 Stunden bei Raumtemperatur gerührt und filtriert. Der Rückstand wird getrocknet und das Filtrat wird vom Lösungsmittel befreit.
   Die Kristalle werden in 5 ml Methylenchlorid aufgenommen und zweimal mit je ca. 3 ml 1N NaOH-Lösung extrahiert. Die organische Phase wird mit 1N HCl, 1N NaOH und gesättigter NaCl-Lösung extrahiert und mit MgSO₄ getrocknet.
   Ausbeute Kristalle nach Aufarbeitung: 174 mg
   Enantiomerenreinheit: 5 % e.e. (nach HPLC)

### C) Herstellung der Katalysator-Komplexe

1) [Iodo-Ru-cym-((+)-5,5'-Dichloro-6,6'-dimethoxy-biphenyl-2,2'-diyl)-bis-diphenylphosphin]iodid
   Eine Lösung von 42 mg (cym₂Ru₂I₄) in 5 ml Methanol/Methylenchlorid (1:1) wird zu einer Mischung von 61 mg ((+)-5,5'-Dichloro-6,6'-dimethoxybiphenyl-2,2'-diyl)-bis-diphenylphosphin in 5 ml Methanol/Methylenchlorid (1:1) gegeben, es wird 10 Minuten unter Luftausschluß unter Rückfluß gekocht und eingeengt.
   ³¹P-NMR (CDCl₃): 41,3 (d, J=61,5 ppm), 25,2 (d, J=61,5 ppm)
2) [(+)-(5,5'-Dichloro-6,6'-dimethoxy-biphenyl-2,2'-diyl)-bis-diphenylphosphin)₂Ru₂Cl₄] NEt₃
   Eine Mischung aus 23,5 mg Dichloro-cycloocta-1,5-dien-ruthenium-(II), 61 mg((+)-5,5'-Dichloro-6,6'-dimethoxy-biphenyl-2,2'-diyl)-bis-diphenylphosphin, 0,035 ml Triethylamin und 3 ml Toluol werden 10 Stunden bei 120°C unter Luftausschluß gerührt. Anschließend wird das Lösungsmittel im Hochvakuum entfernt.
   Ausbeute: quantitativ
   ³¹P-NMR (CDCl₃): 29,1 ppm (s)

### D) Anwendungsbeispiele

1) Hydrierung von 2-(3-Benzyl-phenyl)-propensäure
   Eine Lösung von 1 g 2-(3-Benzyl-phenyl)-propensäure und 460 mg Triethylamin in 15 ml entgastem Methanol wird unter Luftausschluß mit 42,3 mg des in Beispiel C2) hergestellten Katalysators versetzt. Anschließend wird 72 Stunden bei 90 atm bei Raumtemperatur hydriert.
   Ausbeute: quantitativ
   Enantiomerenüberschuß: 88 %
   (Die Bestimmung des Enantiomerenüberschusses erfolgt nach Oxidation zu 2-((3-Benzoyl-phenyl)-propionsäure durch HPLC an einer chiralen Phase wie in EP-A-529 444 beschrieben. Die Herstellung des Eduktes ist ebenfalls in EP-A-529 444 beschrieben.)
2) Hydrierung von Acetessigsäuremethylester
   Eine Lösung von 820 mg Acetessigsäuremethylester in 15 ml entgastem Methanol/Methylenchlorid 1:1 wird unter Luftausschluß mit 33,8 mg des in Beispiel Cl) hergestellten Katalysators versetzt. Anschließend wird 72 Stunden bei 90 atm bei Raumtemperatur hydriert.
   Ausbeute: quantitativ
   Enantiomerenüberschuß: 97 %
   (Die Bestimmung des Enantiomerenüberschusses erfolgt über die Bildung des Mosheresters und anschließende Gaschromatographie-Analytik)

## Patentansprüche

1. Enantiomerenreine Bisphosphine der allgemeinen Formel (I) 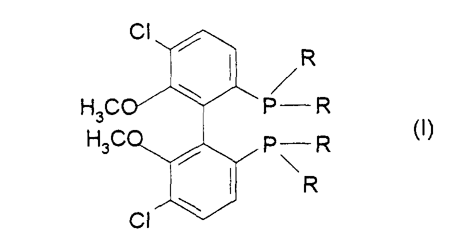 in welcher
R jeweils für eine Phenylgruppe steht, die ihrerseits mit 1 bis 3 Substituenten aus der Gruppe OR¹, R¹, Nitro, NH₂, NHR¹ oder NR¹₂ substituiert sein kann, wobei R¹ eine Alkylgruppe mit bis zu 6 Kohlenstoffatomen darstellt, oder
R eine Alkylgruppe mit bis zu 7 C-Atomen oder eine Cyclo-Alkylgruppe mit 3 bis 7 C-Atomen darstellt.

2. Bisphosphine der allgemeinen Formel (I) gemäß Anspruch 1, in welcher
R jeweils für eine Phenylgruppe steht, die ihrerseits mit 1 bis 3 Substituenten aus der Gruppe OR¹, R¹, Nitro, NH₂, NHR¹ oder NR¹₂ substituiert sein kann, wobei R¹ eine Alkylgruppe mit bis zu 4 Kohlenstoffatomen darstellt, oder
R eine Alkylgruppe mit 1-4 C-Atomen oder eine Cycloalkylgruppe mit 5 oder 6 C-Atomen darstellt.

3. Bisphosphine der allgemeinen Formel (I) gemäß Anspruch 1, in welcher
R für Phenyl steht.

4. Komplexe von Bisphosphinen der allgemeinen Formel (I) gemäß Anspruch 1 mit einem Metall der Gruppe VIII.

5. Komplexe von Bisphosphinen der allgemeinen Formel (I) gemäß Anspruch 1 mit einem Metall aus der Gruppe Rh, Ru oder Ir.

6. Verfahren zur Herstellung von Bisphosphinen der allgemeinen Formel (I) gemäß Anspruch 1 **dadurch gekennzeichnet, daß** man
a) 5-Br-2-Cl-Anisol mit Phosphoroxychloriden der allgemeinen Formel (II) 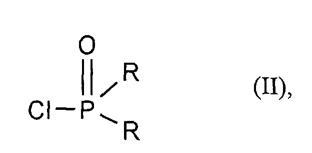 in welcher
R die in Anspruch 1 angegebene Bedeutung hat
nach einer selektiven Mono-Metallierung von Brom-Chlor-Anisol nach üblichen Methoden, zu Verbindungen der allgemeinen Formel (III) 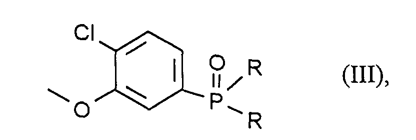 in welcher
R die oben angegebene Bedeutung hat,
umsetzt und diese in 6-Position metalliert und anschließend mit Iod zu Verbindungen der allgemeinen Formel (IV) 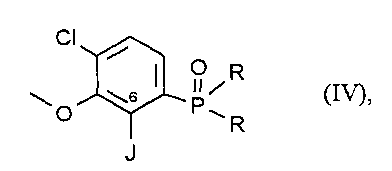 in welcher
R die oben angegebene Bedeutung hat,
umsetzt und anschließend nach üblichen Methoden zu racemischen Verbindungen der allgemeinen Formel (V) 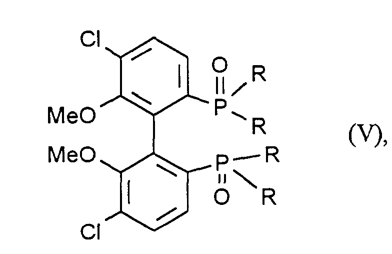 in welcher
R die oben angegebene Bedeutung hat,
dimerisiert und diese Phosphinoxide durch Kristallisation mit enantiomerenreinen Mono- oder Dicarbonsäuren in ihre Enantiomere auftrennt und die getrennten Enantiomere zu Verbindungen der Formel (I) reduziert, oder
b) indem man alternativ Verbindungen der allgemeinen Formel (III) herstellt, indem man 5-Br-2-Cl-Anisol metalliert und mit Phosphinchloriden der allgemeinen Formel (VI) 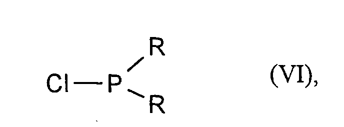 in welcher
R die oben angegebene Bedeutung hat,
umsetzt und anschließend nach üblichen Methoden oxidiert.

7. Verfahren gemäß Anspruch 6, **dadurch gekennzeichnet, daß** man 5-Br-2-Cl-Anisol nach Überführung in eine Mono-Grignard-Verbindung mit Phosphoroxychloriden der allgemeinen Formel (II) 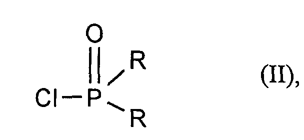 zu Verbindungen der allgemeinen Formel (III) umsetzt und diese mit einem Lithiumamid in 6-Position metalliert und anschließend mit Iod zu Verbindungen der allgemeinen Formel (IV) umsetzt und diese dann über eine Ullmann-Kupplung zu racemischen Verbindungen der allgemeinen Formel (V) dimerisiert und die erhaltenen Phosphinoxide durch Kristallisation mit enantiomerenreinen Weinsäuren in ihre Enantiomere auftrennt und diese dann zu Verbindungen der Formel (I) reduziert.

8. Verwendung von Bisphosphinen der allgemeinen Formel (I) gemäß Anspruch 1 zur Herstellung von Komplexen mit einem Metall der Gruppe VIII.

9. Verwendung von Komplexen gemäß Anspruch 4 bei der asymmetrischen Hydrierung von Ketogruppen zu den entsprechenden Alkoholen oder von C-C-Doppelbindungen.

10. Verwendung von Komplexen gemäß Anspruch 4 bei der asymmetrischen Hydrierung von substituierten oder unsubstituierten α- oder β-Keto-Estern oder -Amiden oder α- oder β-Amino- oder Hydroxy-Ketonen oder der Hydrierung der C-C-Doppelbindung von Allylaminen, Allylalkoholen, Acrylsäuren oder Acetamidozimtsäurederivaten.

11. Verwendung von Komplexen gemäß Anspruch 4 bei der asymmetrischen Hydrierung von 2-Arylpropensäuren oder deren Salzen.

## Claims

1. Enantiomerically pure bisphosphines of the general formula (I) 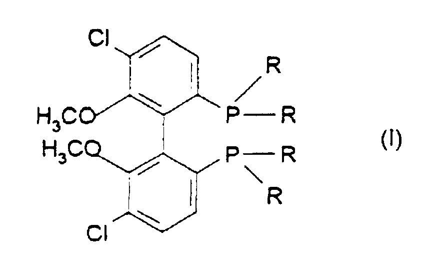 in which
R represents in each case a phenyl group which may in turn be substituted by from 1 to 3 substituents selected from the group consisting of OR¹, R¹, nitro, NH₂, NHR¹ and NR¹₂, where R¹ is an alkyl group having up to 6 carbon atoms, or

2. Bisphosphines of the general formula (I) according to Claim 1, in which
R represents in each case a phenyl group which may in turn be substituted by from 1 to 3 substituents selected from the group consisting of OR¹, R¹, nitro, NH₂, NHR¹ and NR¹₂, where R¹ is an alkyl group having up to 4 carbon atoms, or
R is an alkyl group having from 1 to 4 carbon atoms or a cycloalkyl group having 5 or 6 carbon atoms.

3. Bisphosphines of the general formula (I) according to Claim 1,
in which
R represents pheynyl.

4. Complexes of bisphosphines of the general formula (I) according to Claim 1 with a metal of group VIII.

5. Complexes of bisphosphines of the general formula (I) according to Claim 1, with a metal selected from the group consisting of Rh, Ru and Ir.

6. Process for preparing bisphosphines of the general formula (I) according to Claim 1, **characterized in that**
a) 5-Br-2-Cl-anisole is reacted with phosphorus oxychlorides of the general formula (II) 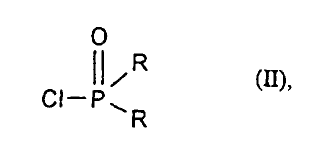 in which 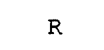
R is as defined in Claim 1,
after a selective monometallation of bromo-chloroanisole by customary methods, to form compounds of the general formula (III) 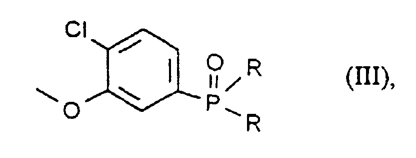 in which
R is as defined above,
and this is metallated in the 6 position and subsequently reacted with iodine to form compounds of the general formula (IV) 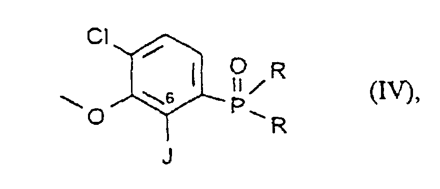 in which
R is as defined above,
and subsequently dimerized by customary methods to form racemic compounds of the general formula (V) 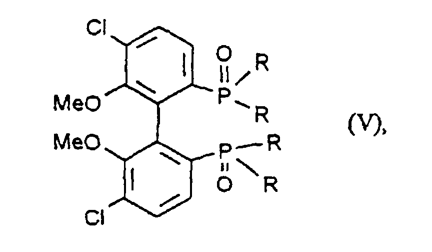 in which
R is as defined above,
and these phosphine oxides are separated into their enantiomers by crystallization with enantiomerically pure monocarboxylic or dicarboxylic acids and the separated enantiomers are reduced to give compounds of the formula (I), or
b) as an alternative, compounds of the general formula (III) are prepared by metallating 5-Br-2-Cl-anisole and reacting this with phosphine chlorides of the general formula (VI) 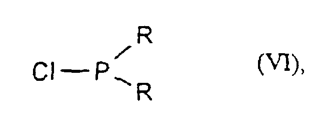 in which
R is as defined above,
and are subsequently oxidized by customary methods.

7. Process according to Claim 6, **characterized in that**
5-Br-2-Cl-anisole is reacted, after conversion into a mono-Grignard compound, with phosphorus oxychlorides of the general formula (II) 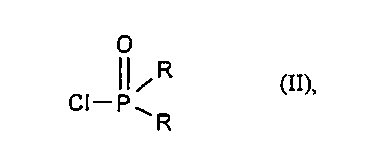 to form compounds of the general formula (III) and these are metallated in the 6 position using a lithium amide and subsequently reacted with iodine to form compounds of the general formula (IV) and these are then dimerized by means of an Ullmann coupling to form racemic compounds of the general formula (V) and the phosphine oxides obtained are separated into their enantiomers by crystallization with enantiomerically pure tartaric acids and these enantiomers are then reduced to give compounds of the formula (I).

8. Use of bisphosphines of the general formula (I) according to Claim 1 for preparing complexes with a metal of group VIII.

9. Use of complexes according to Claim 4 in the asymmetric hydrogenation of keto groups to form the corresponding alcohols or of C-C double bonds.

10. Use of complexes according to Claim 4 in the asymmetric hydrogenation of substituted or unsubstituted α- or β-keto esters or amides or α- or β-amino or hydroxy ketones or the hydrogenation of the C-C double bond of allylamines, allyl alcohols, acrylic acids or acetamidocinnamic acid derivatives.

11. Use of complexes according to Claim 4 in the asymmetric hydrogenation of 2-arylpropenoic acids or their salts.

## Revendications

1. Bisphosphines à l'état d'énantiomères purs, répondant à la formule générale (I) 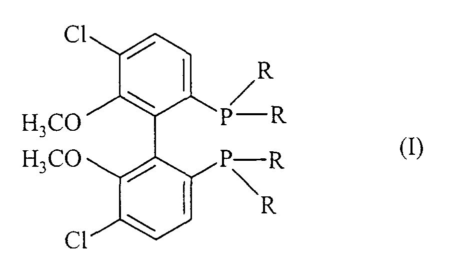 dans laquelle
chacun des symboles R représente un groupe phényle qui peut lui-même porter 1 à 3 substituants choisis parmi OR¹, R¹, les groupes nitro, NH₂, NHR¹ et NR¹₂, R¹ représentant un groupe alkyle contenant jusqu'à 6 atomes de carbone, ou bien
R représente un groupe alkyle contenant jusqu'à 7 atomes de carbone ou un groupe cycloalkyle contenant 3 à 7 atomes de carbone.

2. Bisphosphines de formule générale (I) selon la revendication 1, pour lesquelles
chacun des symboles R représente un groupe phényle qui peut lui-même porter 1 à 3 substituants choisis parmi OR¹, R¹, les groupes nitro, NH₂, NHR¹ et NR¹₂, R¹ représentant un groupe alkyle contenant jusqu'à 4 atomes de carbone, ou bien
R représente un groupe alkyle en C₁-C₄ ou un groupe cycloalkyle en C₅ ou C₆.

3. Bisphosphines de formule générale (I) de la revendication 1, dans laquelle
R représente un groupe phényle.

4. Complexes des bisphosphines de formule générale (I) de la revendication 1 et d'un métal du groupe VIII.

5. Complexes des bisphosphines de formule générale (I) de la revendication 1 et d'un métal du groupe formé par Rh. Ru et Ir.

6. Procédé de préparation des bisphosphines de formule générale (I) de la revendication 1, **caractérisé en ce que**
a) on convertit le 5-Br-2-Cl-anisole, à l'aide d'oxychlorures de phosphore de formule générale (II) 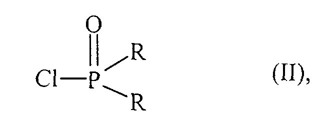 dans laquelle
R a les significations indiquées dans la revendication 1,
après une mono-métallisation sélective du bromo-chloro-anisole, et selon des modes opératoires usuels, en composés de formule générale (III) 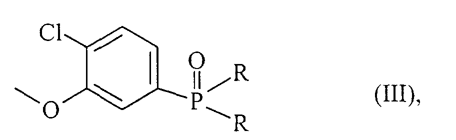 dans laquelle
R a les significations indiquées ci-dessus,
qu'on métallise en position 6 et qu'on fait ensuite réagir avec l'iode, ce qui donne des composés de formule générale (IV) 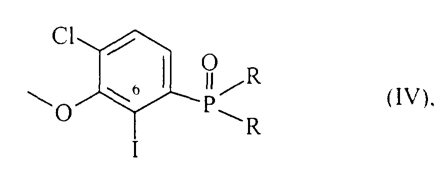 dans laquelle
R a les significations indiquées ci-dessus,
qu'on dimérise ensuite, par des modes opératoires usuels, en composés racémiques de formule générale (V) 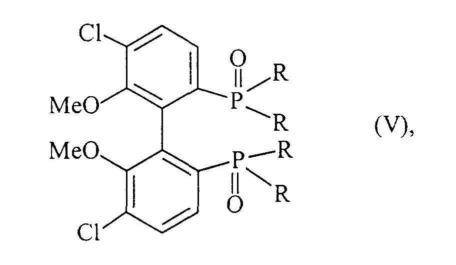 dans laquelle
R a les significations indiquées ci-dessus,
on sépare ces oxydes de phosphine en leurs énantiomères par cristallisation avec des acides mono- ou di-carboxyliques à l'état d'énantiomères purs, et on réduit les énantiomères séparés en composés de formule (I), ou bien
b) dans une variante, on prépare les composés de formule générale (III) par métallisation du 5-Br-2-Cl-anisole et réaction avec des chlorures de phosphines de formule générale (VI) 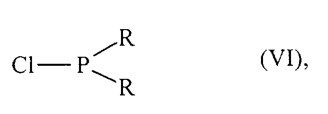 dans laquelle
R a les significations indiquées ci-dessus,
après quoi on oxyde par des modes opératoires usuels.

7. Procédé selon la revendication 6, **caractérisé en ce que** après conversion du 5-Br-2-Cl-anisole en un dérivé de mono-Grignard, on forme par réaction avec des oxychlorures de phosphore de formule générale (II) 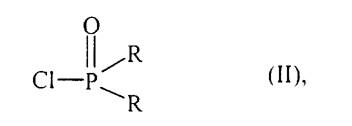 des composés de formule générale (III) qu'on métallise en position 6 par un amidure de lithium, on fait ensuite réagir avec l'iode, ce qui donne des composés de formule générale (IV) qu'on dimérise par une condensation d'Ullmann en composés racémiques de formule générale (V), lesquels oxydes de phosphine sont séparés en leurs énantioméres par cristallisation avec des acides tartriques à l'état d'énantiomères purs, puis on réduit ces énantiomères en composés de formule (I).

8. Utilisation des bisphosphines de formule générale (I) de la revendication 1, pour la préparation de complexes d'un métal du groupe VIII.

9. Utilisation de complexes selon la revendication 4 pour l'hydrogénation asymétrique des groupes céto en les alcools correspondants ou des doubles liaisons C-C.

10. Utilisation des complexes selon la revendication 4 pour l'hydrogénation asymétrique d'α- ou β-céto-esters ou -amides substitués ou non ou d'α- ou β-amino- ou hydroxy-cétones ou pour l'hydrogénation de la double liaison C-C des allylamines, des alcools allyliques, des acides acryliques ou des dérivés de l'acide acétamidocinnamique.

11. Utilisation des complexes de la revendication 4 pour l'hydrogénation asymétrique des acides 2-arylpropénoïques ou de leurs sels.
